(19) Europäisches Patentamt

European Patent Office

Office européen des brevets

(11) Veröffentlichungsnummer: **0 004 571**
**B1**

# (12) EUROPÄISCHE PATENTSCHRIFT

(45) Veröffentlichungstag der Patentschrift:
30.12.81

(21) Anmeldenummer: **79100711.5**

(22) Anmeldetag: **09.03.79**

(51) Int. Cl.³: **C 07 D 249/08, C 08 G 18/80**

(54) **1,2,4-Triazol-blockiertes Isophorondiisocyanat und Biuretgruppen oder Urethangruppen aufweisende Derivate des Isophorondiisocyanats mit 2 bis 4 1,2,4-triazol-blockierten Isocyanatgruppen und Pulverlackbindemittel, die diese Derivate als Vernetzer enthalten.**

(30) Priorität: **21.03.78 DE 2812252**

(43) Veröffentlichungstag der Anmeldung:
**17.10.79 Patentblatt 79/21**

(45) Bekanntmachung des Hinweises auf die Patenterteilung:
**30.12.81 Patentblatt 81/52**

(84) Benannte Vertragsstaaten:
**DE FR GB IT NL**

(56) Entgegenhaltungen:
**US-A-3 721 645**

(73) Patentinhaber: **BAYER AG, Zentralbereich Patente, Marken und Lizenzen, D-5090 Leverkusen 1, Bayerwerk (DE)**

(72) Erfinder: **Wegner, Christian, Dr., Roggendorfstrasse 65, D-5000 Köln 80 (DE)**
Erfinder: **Müller, Hanns-Peter, Dr., Berta-von-Suttner-Strasse 40, D-5090 Leverkusen (DE)**
Erfinder: **Kreuder, Hans Joachim, Dr., Doerperhofstrasse 35, D-4150 Krefeld (DE)**

**1,2,4-Triazol-blockiertes Isophorondiisocyanat und Biuretgruppen oder Urethangruppen aufweisende Derivate des Isophorondiisocyanats mit 2 bis 4 1,2,4-Triazol-blockierten Isocyanatgruppen und Pulverlackbindemittel, die diese Derivate als Vernetzer enthalten**

Die vorliegende Erfindung betrifft 1,2,4-Triazol-blockiertes Isophorondiisocyanat und Biuretgruppen oder Urethangruppen aufweisende Derivate des Isophoron-diisocyanats mit 2 bis 3 1,2,4-Triazol-blockierten Isocyanatgruppen sowie Pulverlackbindemittel, welche diese blockierten Polyisocyanate als Vernetzer enthalten.

Die Herstellung von blockierten Polyisocyanaten ist bekannt (vgl. z.B. Houben-Weyl, Methoden der organischen Chemie, Band XIV/2, 4. Auflage, Georg Thieme Verlag, Stuttgart 1963, Seiten 61–70). Von den zahlreichen in der Literatur beschriebenen Blockierungsmitteln für organische Polyisocyanate haben nur wenige technische Bedeutung erlangt. Für lösungsmittelhaltige Einbrennlacke ist die Verkappung mit Malonsäure- und Acetessigsäureestern gebräuchlich (z.B. DE-PS 756 058), während für Polyurethan-Pulverlackvernetzer nach den Verfahren des Standes der Technik Isophorondiisocyanat bzw. seine NCO-haltigen Derivate im allgemeinen mit Lactamen oder Phenolen blockiert werden (z.B. DE-AS 1 957 483 und DD-PS 55 820).

Keine dieser Verbindungen erfüllt jedoch die an Verkappungsmittel für Pulverlackisocyanate gestellten Forderungen in idealer Weise. Bei Malon- und Acetessigestern ist zwar die Rückspalttemperatur niedrig (120–130 °C/30 Minuten); aufgrund ihrer relativ hohen Molekulargewichte werden jedoch grosse Gewichtsmengen dieser Substanzen benötigt, oder – anders ausgedrückt – der Gewichtsanteil der verkappten NCO-Gruppen wird deutlich herabgesetzt. Ausserdem ist die Vergilbungsresistenz beim Einbrennen bei solchen Systemen oft nicht gegeben. Der Einsatz von Phenolen als Blockierungsmittel ist aufgrund der physiologischen Eigenschaften und der Geruchsbelästigung dieser Verbindungen begrenzt, während bei Anwendung von ε-Caprolactam als Blockierungsmittel zur vollständigen Vernetzung hohe Einbrenntemperaturen (mindestens ca. 170°C/30 Minuten) angewandt werden müssen.

Es wurde nun überraschenderweise gefunden, dass die Eigenschaften der Pulverlackbindemittel des Standes der Technik noch weiter verbessert werden, wenn als Vernetzer 1,2,4-Triazol blockiertes Isophorondiisocyanat bzw. dessen Biuretgruppen oder Urethangruppen aufweisende Derivate enthaltend 2 bis 4 1,2,4-Triazol-blockierte Isocyanatgruppen, vorzugsweise mit einem Schmelzpunkt zwischen 50 und 220 °C, und einem mittleren Molekulargewicht von 306 bis 1000, eingesetzt werden.

In der US-PS 3 248 398 wird die Blockierung von Monoisocyanaten mit H-N-Gruppierungen aufweisenden heterocyclischen Verbindungen beschrieben, wobei die blockierten Monoisocyanate zur Imprägnierung von Textilien und Papier empfohlen werden. Unter der langen Liste der aufgezählten heterocyclischen Verbindungen findet sich auch 1,2,4-Triazol. Hieraus kann jedoch keine Anregung hergeleitet werden, dass mit diesem speziellen Triazol blockierte bestimmte, in dieser Druckschrift nicht erwähnte Polyisocyanate besondere Eigenschaften aufweisen, aufgrund derer sie insbesondere als Vernetzer für Polyurethan-Pulverlacke geeignet sind.

Gemäss der US-PS 3 721 645 können NCO-Gruppen aufweisende Präpolymere, die mit Triazolen, z.B. 1,2,4-Triazol, umgesetzt worden sind, als Überzugsmittel, Spritzgussharze, PVC-Zusätze, Rotationsgussharze und auch als Wirbelsinterpulver (Sp. 3, Z. 63–67; Sp. 4, Z. 3) eingesetzt werden. Sie weisen den Vorteil auf, bereits bei relativ niedrigen Temperaturen auszuhärten (Sp. 4, Z. 5–9, 30–35). Besonders geeignete Ausgangsverbindungen sollen Wasserstoff-aktive Verbindungen mit einem Molekulargewicht von 1000 aufwärts sein (Sp. 3, Z. 59–63). Die Umsetzung von Polyisocyanat, Wasserstoff-aktiver Verbindung und Triazol kann nach der Präpolymer-Methode oder im Eintopfverfahren erfolgen (Sp. 2, Z. 38–44).

Die Produkte, die gemäss US-PS 3 721 645 erhältlich sind, genügen jedoch nicht den Anforderungen, die an hochwertige Pulverlackbindemittel gestellt werden; man verlangt nämlich unter anderem von solchen Produkten, dass sie bei Raumtemperatur gemahlen werden können und dass das gemahlene Pulver bei Temperaturen von 45–50°C über mehrere Monate unter Erhalt der Rieselfähigkeit gelagert werden können. Dies erfordert wiederum einen Schmelzpunkt von Vernetzer und polymerer, OH-funktioneller Komponente von mindestens 70°C. Diese Bedingungen werden von den in der US-PS 3 721 645 beschriebenen Wirbelsinterpulvern nicht erfüllt. Wenngleich die dort beschriebenen Präparate als kleine Brocken oder Granulate bei Raumtemperatur lagerfähig sind, so müssen die Substanzen beim Zermahlen gekühlt werden (Sp. 5, Z. 8; Sp. 6, Z. 48). Dies ist nicht nur zum Erlangen eines hinreichenden Zerkleinerungsgrades notwendig, sondern auch zum Erhalt der Rieselfähigkeit, da bei Temperaturen von 50°C schon eine Vernetzung eintreten (Sp. 4, Z. 32) kann.

Gegenstand der Erfindung sind daher 1,2,4-Triazol-blockiertes Isophorondiisocyanat und Biuretgruppen oder Urethangruppen aufweisende Derivate des Isophorondiisocyanats mit 2 bis 4 1,2,4-Triazol-blockierten Isocyanatgruppen, vorzugsweise mit einem Schmelzpunkt von 50 bis 220°C, insbesondere 70 bis 200°C, dadurch gekennzeichnet, dass sie ein mittleres Molekulargewicht von 306 bis ca. 1000 aufweisen.

Das mittlere Molekulargewicht wird dampfdruckosmometrisch in Aceton als Lösungsmittel bestimmt.

Weiterer Gegenstand der Erfindung sind Pulverlackbindemittel auf Basis von Polyhydroxylverbindungen, die diese 1,2,4-Triazol-blockierten Verbindungen als Vernetzer enthalten.

Als Ausgangsmaterial zur Herstellung der erfindungsgemässen Verbindungen dient das Isophorondiisocyanat selbst sowie seine Urethan-, oder Biuretgruppen aufweisenden Derivate mit 2-4 freien Isocyanatgruppen. Bevorzugt sind urethangruppenhaltige Isocyanate, die durch Umsetzung des Isophorondiisocyanats mit unterschüssigen Mengen an aliphatischen Diolen oder Triolen des Molekulargewichtsbereiches 62–200, wie z.B. Ethylenglykol, 1,2-Propandiol, 1,3-Propandiol, 1,2-Butandiol, Tetramethylenglykol, Hexamethylenglykol, Diethylenglykol, Trimethylolpropan oder Glycerin entstehen. Bei der Herstellung dieser Urethan-modifizierten Derivate der obengenannten Diisocyanate werden die Diisocyanate mit den beispielhaft genannten mehrwertigen Alkoholen in Mengenverhältnissen zur Reaktion gebracht, die einem NCO/OH-Äquivalentverhältnis von mindestens 2:1, vorzugsweise 2:1 bis 20:1 entsprechen. Auch die so erhältlichen Urethan-modifizierten Polyisocyanate weisen NCO-Funktionalitäten von 2 bis 4, vorzugsweise 2 bis 3, und (mittlere) Molekulargewichte von unter 850 auf.

Reaktionspartner für die beispielhaft genannten Polyisocyanate zur Herstellung der erfindungsgemäss blockierten Polyisocyanate ist das erfindungswesentliche Blockierungsmittel 1,2,4-Triazol. Das erfindungswesentliche Blockierungsmittel wird vorzugsweise als alleiniges Blockierungsmittel verwendet. Denkbar ist jedoch auch die gleichzeitige Mitverwendung anderer an sich bekannter Blockierungsmittel, wie z.B. ε-Caprolactam oder beliebiger Phenole.

Die Umsetzung der Ausgangspolyisocyanate mit dem Blockierungsmittel kann in Gegenwart aprotischer, d.h. gegenüber Isocyanatgruppen, inerter Lösungsmittel oder in der Schmelze erfolgen. Geeignete Lösungsmittel sind z.B. Äthylacetat, Butylacetat, Aceton, Methyläthylketon, Methylisobutylketon, Tetrahydrofuran oder Dioxan. Die Blockierungsreaktion erfolgt im allgemeinen im Temperaturbereich von 40 bis 160, vorzugsweise 60 bis 130°C. Bei Reaktionstemperaturen oberhalb 60°C kann die Umsetzung in Abwesenheit von Katalysatoren durchgeführt werden und ist in den meisten Fällen bereits nach 30-60 Minuten beendet. Das Blockierungsmittel wird vorzugsweise in mindestens äquivalenter Menge eingesetzt. Oft empfiehlt sich zur vollständigen Verkappung ein geringer Überschuss. Bei der erwähnten, jedoch nicht bevorzugten Mitverwendung anderer Blockierungsmittel wird das 1,2,4-Triazol auf jeden Fall in solchen Mengen mitverwendet, dass in den erfindungsgemässen blockierten Polyisocyanaten pro Molekül mindestens zwei 1,2,4-Triazol-blockierte Isocyanatgruppen vorliegen.

Bei Verwendung der beispielhaft genannten Urethan-modifizierten Polyisocyanate als Ausgangsmaterial kann deren Herstellung sowie deren Blockierung in einer einstufigen Reaktion erfolgen und zwar dergestalt, dass man das nicht urethanmodifizierte Diisocyanat mit einem Gemisch aus Blockierungsmittel und einem oder mehreren der beispielhaft genannten Polyole zur Reaktion bringt. Auch bei dieser Ausführungsform der Herstellung der erfindungsgemässen blockierten Polyisocyanate gelten die oben gemachten Ausführungen bezüglich der Mengenverhältnisse der Reaktionspartner, wobei die Angaben bezüglich des Verhältnisses zwischen NCO-Gruppen und NH-Gruppen sich selbstverständlich nur auf diejenigen NCO-Gruppen beziehen, die nicht für die Reaktion mit den Hydroxylgruppen benötigt werden.

Bei der Herstellung der erfindungsgemässen Produkte kann sowohl das Blockierungsmittel vorgelegt und das zu blockierende Polyisocyanat in das vorgelegte Blockierungsmittel gegeben als auch umgekehrt verfahren werden, dergestalt, dass das Blockierungsmittel gegebenenfalls zusammen mit dem Alkohol zur Modifizierung des Ausgangsisocyanats dem vorgelegten Ausgangsisocyanat zudosiert wird. Bei Verwendung als Gemisch aus Polyol und Triazol zur Herstellung der erfindungsgemässen Verbindungen ist es besonders vorteilhaft, dieses Gemisch durch Erwärmen in eine klare Schmelze umzuwandeln, die dann mittels eines dampfbeheizten Tropftrichters zu dem Isocyanat dosiert wird.

Bei der Herstellung der erfindungsgemässen Verbindungen in Gegenwart eines Lösungsmittels für die Ausgangsmaterialien können die blockierten Polyisocyanate auch auskristallisieren und können dann durch einfaches Filtrieren als Festsubstanz gewonnen werden.

Im allgemeinen weisen aus definierten Ausgangsmaterialien hergestellte erfindungsgemässe Verbindungen ein genau definiertes Molekulargewicht und einen scharfen Schmelzpunkt auf. Bei Verwendung von Polyisocyanatgemischen und/oder von Gemischen aus verschiedenen Blockierungsmitteln, insbesondere bei Verwendung der beispielhaft genannten Biuret- oder Urethan-modifizierten Ausgangsisocyanate zur Herstellung der erfindungsgemässen blockierten Polyisocyanate handelt es sich bei letzteren um Gemisch verschiedener Verbindungen des angegebenen mittleren Molekulargewichts.

Die erfindungsgemässen blockierten Polyisocyanate stellen wertvolle Vernetzer für Polyhydroxylverbindungen dar und eignen sich insbesondere in Kombination mit Polyhydroxylverbindungen eines über 50°C, vorzugsweise zwischen 60 und 120°C, liegenden Erweichungspunkts als Hitze-vernetzbares Zweikomponenten-Bindemittel für Pulverlacke. Hierbei ergeben sich bei Verwendung der erfindungsgemässen blockierten Polyisocyanate insbesondere folgende Vorteile:

1. Wegen des niederen Molekulargewichts des 1,2,4-Triazol werden nur relativ geringe Mengen des Blockierungsmittels zur Blockierung der Isocyanatgruppen benötigt, so dass der Gewichtsanteil des bei der Vernetzung abspaltenden Blockierungsmittels relativ gering bleibt.

2. Das erfindungsgemässe Blockierungsmittel ist physiologisch weit weniger bedenklich als andere Blockierungsmittel des Standes der Technik.

3. Der vergleichsweise hohe Schmelzpunkt der erfindungsgemässen blockierten Polyisocyanate macht sie insbesondere für Überzüge geeignet, die nach dem Wirbelsinter- oder elektrostatischen Pulversprüh-Verfahren (EPS-Verfahren) appliziert und anschliessend einer thermischen Behandlung unterworfen werden. So ist es bei Einsatz des Isophorondiisocyanats als Isocyanatkomponente nicht notwendig, dieses, wie beispielsweise in der DE-AS 2 215 080 beschrieben, unter Verlust von etwa der Hälfte der Isocyanatgruppen zu modifizieren, es genügt vielmehr, nur etwa 15% der Isocyanatgruppen des IPDI durch mehrwertige Alkohole zu verlängern, um eine ausreichende Rieselfähigkeit der Pulver zu gewährleisten.

4. Vorteilhaft ist ferner die vergleichsweise niedrige Einbrenntemperatur von ca. 150°C beim Verarbeiten der erfindungsgemäss blockierte Polyisocyanate als Vernetzer enthaltenden Pulverlacke.

Bei der Verwendung der erfindungsgemässen blockierten Polyisocyanate kommen diese im allgemeinen in solchen Mengen zum Einsatz, dass für jede gegenüber Isocyanatgruppen reaktionsfähige Gruppe des Bindemittels 0,8 bis 1,2, vorzugsweise ca. 1, blockierte Isocyanatgruppe der erfindungsgemässen blockierten Polyisocyanate zur Verfügung steht.

Darüber hinaus wird die Art der erfindungsgemässen blockierten Polyisocyanate und/oder die Art der Komponente mit gegenüber Isocyanatgruppen reaktionsfähigen Gruppen bei der erfindungsgemässen Verwendung so gewählt, dass das gebrauchsfertige Bindemittel einen oberhalb 40°C, vorzugsweise zwischen 40 und 100°C, liegenden Schmelzpunkt bzw. -bereich aufweist, d.h. innerhalb dieses Temperaturbereichs zu einer filmbildenden Flüssigkeit schmilzt.

Reaktionspartner für die erfindungsgemässen blockierten Polyisocyanate bei deren erfindungsgemässen Verwendung sind insbesondere beliebige organische Polyhydroxylverbindungen des bereits obengenannten Schmelz- bzw. Erweichungspunktes. Vorzugsweise werden höhermolekulare Polyhydroxylverbindungen mit Hydroxylzahlen zwischen 40 und 250, vorzugsweise 40 und 150, eingesetzt. Beispiele derartiger Verbindungen sind hydroxylgruppenhaltige Polyester, hydroxylgruppenhaltige Polyäther, hydroxylgruppenhaltige Polyurethane, hydroxylgruppenhaltige Polyesterurethane, hydroxylgruppenhaltige Urethanalkydharze, hydroxylgruppenhaltige Acrylharze, hydroxylgruppenhaltige Epoxidharze sowie deren Gemische. Polyesterpolyole sind bevorzugt. Diese Polyesterpolyole lassen sich nach bekannten Verfahren, vorzugsweise aus cyclischen Polycarbonsäuren, wie Phthalsäure, Isophthalsäure, Terephthalsäure, Benzol-1.2.4-tricarbonsäure, 3,6-Dichlorphthalsäure, Tetrachlorphthalsäure, Tetrahydrophthalsäure, Hexahydrophthalsäure, Endomethylentetrahydrophthalsäure bzw. – soweit zugänglich – deren Anhydriden oder niederen Alkylestern, und Trimellithsäureanhydrid, sowie Diolen, wie Äthylenglykol, 1,2-Propandiol, 1,3-Propandiol, 1,2-Butandiol, 1,4-Butandiol, 2,2-Dimethylpropandiol, Hexandiol-2,5, Hexandiol-1,6, 4,4'-Dihydroxydicyclohexylpropan-2,2, Cyclohexandiol, Dimethylolcyclohexan, Diäthylenglykol und 2,2-Bis-[4-(β-hydroxy-äthoxy)-phenyl]-propan und Polyolen, wie Glycerin, Hexantriol, Pentaerythrit, Sorbit, Trimethyloläthan, Trimethylolpropan und Tris-(β-hydroxyäthyl)-isocyanurat, herstellen.

In geringen Mengen können Monocarbonsäuren, z.B. Benzoesäure, tert.-Butylbenzoesäure, Hexahydrobenzoesäure, gesättigte und/oder ungesättigte Fettsäuren, sowie acyclische Polycarbonsäuren, wie Adipinsäure, Maleinsäure und Bernsteinsäure, einkondensiert werden.

Ebenfalls geeignet, jedoch weniger bevorzugt, sind Polyvinylharze des genannten Hydroxylzahlbereichs, wie sie in an sich bekannter Weise durch Mischpolymerisation geeigneter Monomeren, wie z.B. Hydroxypropyl-(meth)-acrylsäureester, Hydroxy-äthyl-(meth)-acrylsäureester, Äthylacrylat, Butylacrylat, 2-Äthylhexylacrylat, Methylmethacrylat, Äthylmethacrylat, Butylmethacrylat, Laurylmethacrylat, Styrol, α-Methylstyrol, Vinyltoluol, Acrylnitril, Acrylamid, Vinylacetat, Acrylsäure und Methacrylsäure, in Gegenwart von Initiatoren und Reglern hergestellt werden können.

Ebenfalls geeignet, jedoch weniger bevorzugt, sind die an sich bekannten Polyätherpolyole, falls sie den genannten Bedingungen bezüglich des Schmelz- bzw. Erweichungspunkts entsprechen.

Auch Polyepoxidharze, beispielsweise Umsetzungsprodukte von Bisphenol A mit Epichlorhydrin, können in den Pulverlackbindemitteln mitverwendet werden.

Auch übliche Zusatzstoffe wie Pigmente, Verlaufshilfsmittel, Füllstoffe und Katalysatoren, wie sie z.B. in der Deutschen Patentschrift 946 173 genannt sind, können zugesetzt werden.

Die Herstellung der Filme erfolgt in der üblichen Weise. Beide Komponenten sowie die Hilfsstoffe werden beispielsweise auf einem Kollergang innig vermischt und anschliessend in einem Extruder bei 90–120°C homogenisiert. Nach Erkalten wird die Masse auf eine Kornfeinheit von unter 100 μm gemahlen, das Pulver auf einer Pulversprühanlage auf das Substrat aufgetragen und anschliessend eingebrannt.

Die mit 1,2,4-Triazol-verkappten Isocyanaten gehärteten Filme zeigen neben guten Oberflächeneigenschaften sehr gute Lösungsmittelbeständigkeit, Elastizität, Vergilbungsresistenz und Wetterbeständigkeit.

Beispiel 1
Herstellung eines 1,2,4-Triazol-blockierten Isocyanats

888 g Isophorondiisocyanat werden bei 100°C portionsweise mit 580 g 1,2,4-Triazol versetzt. Man rührt 3 Stunden bei 100°C nach und giesst die Schmelze aus, die beim Abkühlen zu einer klaren spröden Masse erstarrt. Schmp. 74°C
NCO (verkappt): 22,9%

Beispiel 2

Herstellung eines 1,2,4-Triazol-blockierten urethangruppenhaltigen Isocyanats

888 g Isophorondiisocyanat und 44,2 g Trimethylolpropan werden bei 90°C 1 Stunde gerührt. Anschliessend gibt man portionsweise 510 g 1,2,4-Triazol so zu, dass die Temperatur nicht über 120°C steigt. Nach 3 Stunden ist kein freies Isocyanat mehr nachzuweisen. Man giesst die klare, farblose Schmelze aus, die beim Abkühlen zu einer klaren spröden Masse erstarrt.
Schmp.: 78°C
NCO (verkappt): 20,3%

Beispiel 3

Herstellung einer Beschichtung nach dem EPS-Verfahren

50,5 Teile eines Polyesters, hergestellt aus Terephthalsäure, Neopentylglykol und Trimethylolpropan (OH-Zahl = 50, Schmp.: 65°C, Säurezahl <10) werden mit 9,0 Teilen des nach Beispiel 2 hergestellten 1,2,4-Triazol-verkappten Isocyanats, 29,9 Teilen Titan-Weisspigment (Rutil) und 0,6 Teilen eines handelsüblichen Verlaufmittels in einem Extruder bei 100–120°C mit einer Verweilzeit von 30–60 sec homogenisiert und anschliessend in einer Mühle auf eine Kornfeinheit von <100 μ gemahlen. Mit einer Spannung von 60 kV wird das Pulver nach dem EPS-Verfahren auf fettfreie Eisenbleche aufgetragen und eingebrannt. Man erhält lösungsmittelbeständige, elastische und lichtechte Überzüge mit folgenden Werten:

| Einbrenn-<br>bedingungen | (Min/°C) | 30/140 | 30/160 | 7/200 |
|---|---|---|---|---|
| Dicke | (nm) | 62–74 | 60–65 | 49–58 |
| Glanz<br>DIN 67 530 | nach<br>Gardner<br>60°C | 89 | 88 | 88 |
| Gitterschnitt<br>DIN 53 151 | | 0 | 0 | 0 |
| Reverse<br>impact | (Joule) | >11.25 | >11.25 | >11.25 |
| Erichsen-<br>tiefung<br>DIN 53 156 | (mm) | 8 | 8 | 8 |

**Patentansprüche**

1. 1,2,4-Triazol-blockiertes Isophorondiisocyanat und Biuretgruppen oder Urethangruppen aufweisende Derivate des Isophorondiisocyanats mit 2 bis 4 1,2,4-Triazol-blockierten Isocyanatgruppen, vorzugsweise mit einem Schmelzpunkt von 50 bis 220°C, dadurch gekennzeichnet, dass sie ein mittleres Molekulargewicht von 306 bis ca. 1000 aufweisen.

2. Pulverlackbindemittel auf Basis von Polyhydroxylverbindungen, dadurch gekennzeichnet, dass sie die Verbindungen nach Anspruch 1 als Vernetzer enthalten.

**Revendications**

1. Isophoronediisocyanate bloqué par le 1,2,4-triazole et dérivés de l'isophoronediisocyanate présentant des groupes biuret ou des groupes uréthanne, ayant 2 à 4 groupes isocyanate bloqués par le 1,2,4-triazole, de préférence d'un point de fusion de 50 à 220°C, caractérisés en ce qu'ils présentent un poids moléculaire moyen de 306 à environ 1000.

2. Liants pour peintures ou vernis en poudre à base de composés polyhydroxylés, caractérisés en ce qu'ils contiennent comme agents réticulants les composés selon la revendication 1.

**Claims**

1. An isophorone diisocyanate blocked with 1,2,4-triazole, and derivatives, containing biuret groups or urethane groups, of the isophorone diisocyanate, containing 2 to 4 isocyanate groups blocked with 1,2,4-triazole, preferably having a melting point of 50 to 220°C, characterised in that they have an average molecular weight of 306 to about 1,000.

2. Powder lacquer binders based on polyhydroxyl compounds, characterised in that they contain the compounds according to Claim 1 as cross-linkers.